# EUROPÄISCHE PATENTSCHRIFT

(11) **EP 1 343 536 B1**
(45) Veröffentlichungstag und Bekanntmachung des Hinweises auf die Patenterteilung: **12.10.2005**
(21) Anmeldenummer: 01271089.3
(22) Anmeldetag: 05.12.2001
(51) Int. Cl.: A61L 2/02, A61L 2/16, A61F 2/24, A61F 2/06, A61L 27/36

(54) **VERFAHREN ZUR DEZELLULARISIERUNG VON FREMDMATERIAL ZUR HERSTELLUNG VON BIOPROTHESEN**
METHOD FOR DECELLULARISING FOREIGN MATERIAL FOR THE PRODUCTION OF BIOPROSTHESES
PROCEDE DE DECELLULARISATION D'UNE MATIERE ETRANGERE POUR LA PRODUCTION DE BIOPROTHESES

(30) Priorität: 20.12.2000 DE 10064948
(43) Veröffentlichungstag der Anmeldung: 17.09.2003
(73) Patentinhaber: Auto Tissue Gmbh, 10115 Berlin (DE)
(72) Erfinder: KONERTZ, Wolfgang, 14195 Berlin (DE); DOHMEN, Pascal, 10115 Berlin (DE)
(74) Vertreter: Wablat, Wolfgang
(86) Internationale Anmeldenummer: PCT/DE2001/004616
(87) Internationale Veröffentlichungsnummer: WO 2002/049681

(56) Entgegenhaltungen:
- WO-A-01/49210
- WO-A-84/01894
- WO-A-96/32905
- WO-A-97/18842
- US-A- 4 315 919
- US-A- 4 546 642
- US-A- 5 846 828

## Beschreibung

Die Erfindung betrifft ein Verfahren zur Dezellularisierung von allogenem und xenogenem Fremdmaterial mit Hilfe biologischer Detergentien für die Herstellung von mit körpereigenen Zellen des Prothesenempfängers beschichteten Bioprothesen.

Für die Herstellung von Bioprothesen aus xenogenem oder allogenem Fremdmaterial, das mit körpereigenen Zellen des späteren Empfängers der Prothese beschichtet wird, ist es zur Vermeidung immunologischer Reaktionen und zur Gewährleistung des Wachstums und der Regeneration der neu angesiedelten körpereigenen Zellen erforderlich, eine von Fremdzellen befreite "azellulare" Struktur zur Verfügung zu stellen. Mit den bekannten Dezellularisierungsverfahren und Verwendung biologischer Detergentien ist jedoch eine vollständige Herauslösung des gesamten Zellmaterials aus der Gewebematrix nicht möglich, so dass virale - bisher noch unbekannte - Wirkungen, beispielsweise der in porcinem Gewebe enthaltenen Viren, nicht auszuschließen sind.
Eine Apparatur für die Sterilisierung und Besiedlung, sowie für die Aufbewahrung von Herzklappen-Implantaten ist lediglich aus dem US-Patent 5,846,828 bekannt. Dieser Apparat verfügt über eine Kammer, welche eine Umgebung ermöglicht, die den physiologischen Bedingungen im menschlichen Herzen entspricht.

Der Erfindung liegt daher die Aufgabe zugrunde, ein Verfahren zur Dezellularisierung von zur Beschichtung mit körpereigenen Zellen vorgesehenem Fremdmaterial anzugeben, dass eine vollständige, aber schonende Entfernung der Zellen aus dem fremden Gewebe gewährleistet.

Erfindungsgemäß wird die Aufgabe mit einem Verfahren gemäß den Merkmalen des Patentanspruches 1 gelöst.

Der Grundgedanke der Erfindung besteht mit anderen Worten darin, dass die Entfernung der Fremdzellen von dem für eine Neubeschichtung mit körpereigenen Zellen vorgesehenen allogenen oder xenogenen Ausgangsprodukt durch die Kombination einer Gallensäurebehandlung mit einer Alkoholbehandlung und zwischen- bzw. nachgeschalteten Spülschritten sowie mit einer mechanischen Einwirkung auf die Gewebematrix und die zu entfernenden Zellen durch ein strömendes Medium mindestens im letzten Spülschritt vorgenommen wird.

Die vorzugsweise in Form von Desoxycholsäure eingesetzte Gallensäure bewirkt eine allmähliche - bei mechanischer Einwirkung beschleunigte - Ummantelung der Zellen mit der Säure, um eine Trennschicht zwischen der Matrix aus Kollagen und Elastin (im folgenden als Kollagen-Matrix bezeichnet) und der Zelle zu schaffen und die Verbindung mit der Matrix zu lösen. Gleichzeitig hat die Desoxycholsäure eine die Zellen abtötende Wirkung. In dem anschließenden Spülschritt werden die abgelösten Zellen weggespült und auch die Desoxycholsäure abgespült. Bei der darauffolgenden Alkoholbehandlung, vorzugsweise in Ethanol oder Propanol, wird gegebenenfalls noch vorhandene Desoxycholsäure, die sich gut in Alkohol löst, restlos entfernt. Mit gegebenenfalls vorhandener Rest-Desoxycholsäure und Alkohol werden in der Matrix noch vorhandene Zellen gelöst, während der Alkohol darüber hinaus eine zellentötende und antivirale Wirkung hat. Der anschließende letzte Spülschritt ist eine - vorzugsweise pulsierende - Strömung, deren Kräfte zum einen auf die Wände des betreffenden Organteils wirken und die Matrix weiten und zum anderen unmittelbar auf noch vorhandene Restzellen mechanisch einwirken und diese aus der geweiteten Matrix herauslösen.

Es können auch andere oder alle Verfahrensschritte durch eine vorzugsweise pulsierende Strömung des betreffenden Mediums mit mechanischen Kraftwirkungen verbunden sein. So hat der oben bereits erwähnte pulsierende Desoxycholsäurestrom aufgrund der Bewegung und Weitung der Matrix eine schnellere Ausbildung der Trennschicht zwischen der Zelle und der Kollagen-Matrix und gleichzeitig ein leichteres Ablösen der Zelle durch die auf diese wirkenden Kräfte zur Folge.

Aus den Unteransprüchen und der unten wiedergegebenen Beschreibung eines Ausführungsbeispiels ergeben sich weitere Merkmale und,vorteilhafte Ausgestaltungen der Erfindung.

Mit dem vorgeschlagenen Verfahren gelingt es, azellulare, das heißt, von jeglichem Zellmaterial und damit von Viren freie Organteile wie Herzklappen oder Gefäße als Ausgangsprodukt für die Herstellung von Bioprothesen durch anschließende Beschichtung mit körpereigenen Zellen des jeweiligen Empfängers bereitzustellen.

Die Vorrichtung zur Behandlung des aus Fremdmaterial bestehenden Organteils in einem strömenden Medium umfasst eine Dezellularisierungskammer zur Aufnahme des betreffenden Organteils und eine Pumpe zur Erzeugung des Medienstroms, die nacheinander in eine Ringleitung eingebunden, sind. In der Ringleitung befinden sich Füll- und Ablassventile zum Zuführen bzw. Ablassen des jeweiligen Behandlungsmediums. Die Dezellularisierungskammer ist lösbar mit der Ringleitung verbunden, so dass diese und damit das in dem Medium befindliche Organteil auch bewegt werden kann. In dem Behälter ist das zu behandelnde Organteil durch Annähen an entsprechend dem Organteil ausgebildeten Einsatzstücken unter Vorspannung längs zur Strömungsrichtung fixiert.

Ein Ausführungsbeispiel der Erfindung wird anhand der Zeichnung näher erläutert. Es zeigen
- Fig. 1: eine Vorrichtung zur Dezellularisierung einer Herzklappe in einem Strömungskreislauf;
- Fig. 2: eine Schnittansicht einer die Herzklappe aufnehmenden, in den Strömungskreislauf eingebundenen Dezellularisierungskammer;

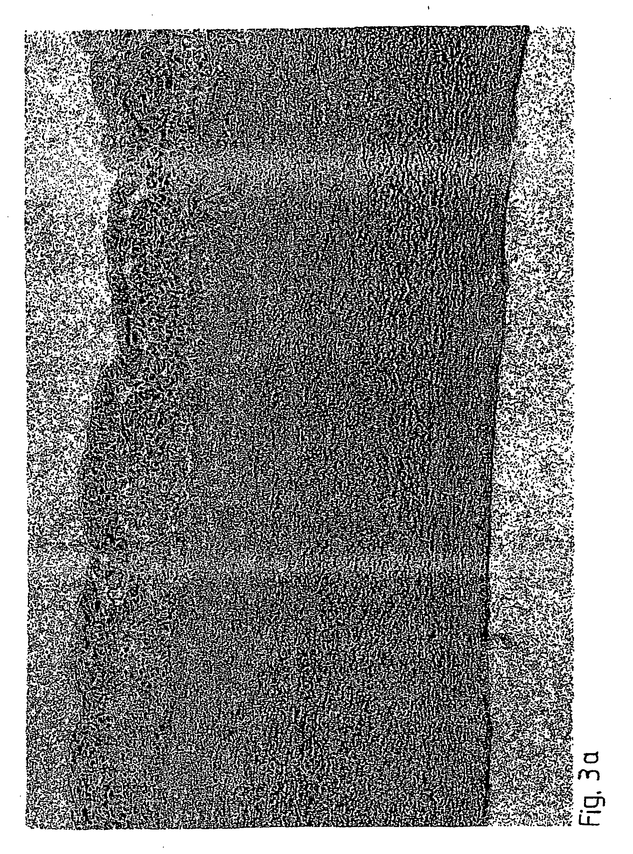

- Fig. 3a: eine mikroskopische Schnittansicht einer nach dem erfinndungsgemäßen Verfahren dezellularisierten Wand einer Aortenklappe; und

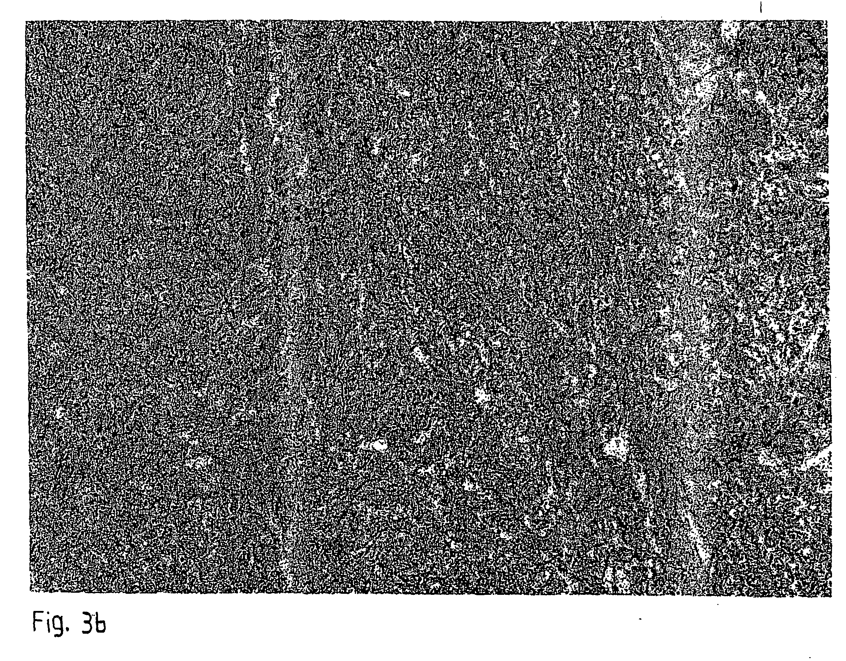

- Fig. 3b: eine vergrößerte Darstellung eines medialen Gewebeabschnittes der Aortenklappenwand nach Fig. 3a.

In dem hier beschriebenen Ausführungsbeispiel wird eine im Schlachthof entnommene porcine Aortenklappe von Fett befreit, zugeschnitten und ausgemessen sowie auf das Vorhandensein von Keimen (Fungi, aerobe und anaerobe Bakterien, Mycoplasma) untersucht. Eine Zwischenlagerung bei max 4°C soll sieben Tage nicht überschreiten.

Die so vorbereitete Herzklappe wird in eine 1 bis 2 %-ige Desoxycholsäurelösung (oder eine Gallensäure mit ähnlicher Wirkung) eingebracht und darin bis zu 24 h bei 37°C gelagert. Die Desoxycholsäure ist in der Lage, mit Fettsäure sogenannte Addukte in Form von Einschlussverbindungen zu bilden, so dass sich die Desoxycholsäure allmählich allseitig an die Zelle anlegen kann und deren Haftverbindung mit der Gewebematrix somit gelöst wird. Die Desoxycholsäure hat gleichzeit eine die Zellen abtötende Wirkung.

Anschließend wird die so behandelte Herzklappe in einer Verdünnungsreihe aus Phosphatpufferlösung (PBS) mit immer geringer werdender Konzentration bei gleichzeitiger Bewegung der Herzklappe gespült, um die mit der Desoxycholsäure behandelten Zellen von der Gewebematrix zu entfernen.

In dem darauffolgenden dritten Schritt wird die Herzklappe bei Raumtemperatur etwa 10 Minuten in 40 %-igem Alkohol behandelt, um dadurch eine antivirale Wirkung zu erzielen und in dem Kollagengerüst gegebenenfalls noch vorhandene Zellen weiter abzutöten. Da Alkohol ein gutes Lösungsmittel ist, werden gleichzeitig eventuell noch vorhandene Reste der Säure ausgespült und weitere Zellen abgelöst.

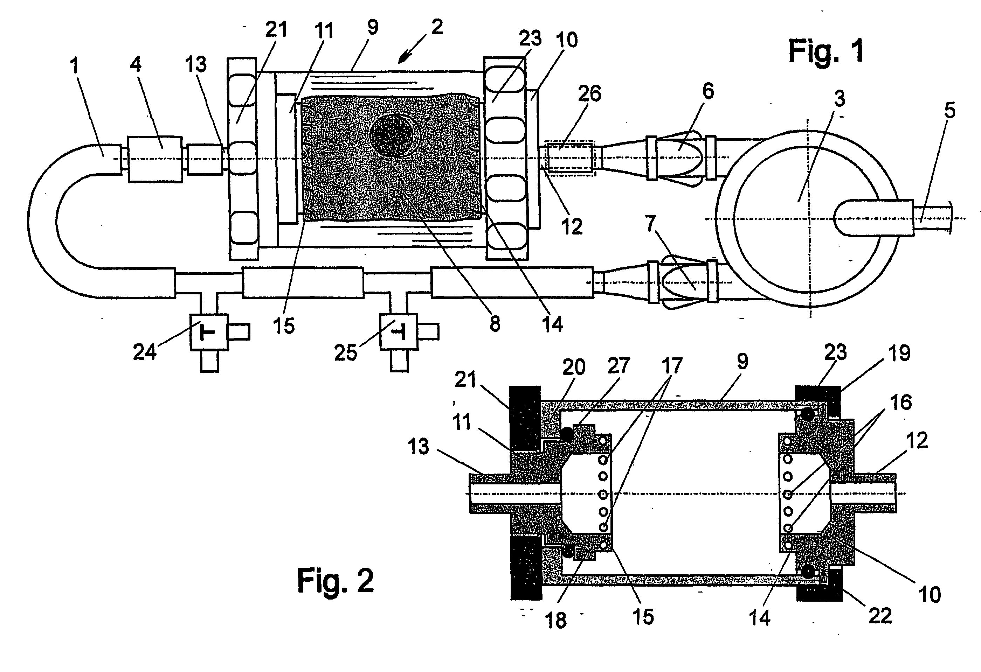

In einer weiteren Verdünnungsreihe aus Phosphatpufferlösung (PBS - Phosphat Buffer Solution) wird die Herzklappe erneut gespült und anschließend in einem pulsierenden PBS-Medienstrom mechanisch behandelt. Durch den pulsierenden Medienstrom wird die in einer Dezellularisierungskammer in Längsrichtung durchströmte und unter Vorspannung fixierte Herzklappe rhythmisch geweitet und gleichzeitig durch die Strömungskräfte mechanisch beaufschlagt. Dadurch werden auch noch die restlichen Zellen mechanisch aus dem Kollagengerüst herausgelöst, so dass letztlich eine azellulare Struktur vorliegt, aus der jegliches Zellmaterial entfernt ist und in der daher auch keine Viren enthalten sein können. Eine so behandelte - zellfreie und auch von den eingesetzten Dezellularisierungsmedien freie Gewebematrix - der Herzklappe, wie sie in Fig. 2 dargestellt ist, eignet sich hervorragend zur Neubeschichtung mit körpereigenen Endothelzellen des späteren Empfängers einer solchen Bioprothese, die ohne die Gefahr immunologischer Reaktionen oder viraler Einflüsse in den menschlichen Körper implantiert werden kann.

Die Erfindung ist sowohl hinsichtlich der Art und Herkunft, des zur Herstellung von Bioprothesen vorgesehenen Fremdmaterial als auch der Verfahrensparameter nicht auf die oben beispielhaft erläuterte Behandlungsvariante beschränkt, sofern die wesentlichen Verfahrensschritte, d.h. Behandlung in einer Addukte bildenden Gallensäure und in Alkohol mit zwischen- bzw. nachgeschaltetem Spülen in Kombination mit einer vorzugsweise pulsierenden Durchströmung des betreffenden Organteils zur schonenden mechanischen Einwirkung auf das Gewebe, durchgeführt werden. So ist es auch möglich, nicht nur den letzten Spülschritt, sondern stattdessen oder zusätzlich andere oder alle Behandlungsstufen in einem strömenden Medium ablaufen zu lassen. Dadurch wird die Wirkung des jeweiligen Mediums mechanisch unterstützt, indem ein besserer, allseitiger Zugang zu den Zellen erreicht wird und die Zellen durch die Kraftwirkung der pulsierenden Strömung leichter aus der geweiteten Kollagen-Matrix herausgelöst bzw. von dieser abgelöst werden.

Eine Vorrichtung zur Dezellularisierung einer Herzklappe ist in Fig. 1 dargestellt. Sie umfasst eine Ringleitung 1, in die eine die zu behandelnde Herzklappe 3 aufnehmende Dezellularisierungskammer 2, eine Membranpumpe 3 und eine der Dezellularisierungskammer unmittelbar nachgeschaltete Ausgleichskammer 4 eingebunden sind. Die Membranpumpe 3 ist über eine Schlauchleitung 5 an eine Antriebseinheit (nicht dargestellt) angeschlossen. In die beiden Anschlüsse der Membranpumpe 3 an die Ringleitung 1 sind ein Auslassventil 6 und ein Einlassventil 7, deren Funktion etwa der einer Herzklappe entspricht, integriert. Auf das Auslassventil 6 kann jedoch bei der Behandlung von Herzklappen wegen der dort bereits vorhandenen Ventilklappen auch verzichtet werden.

Das Kernstück der Vorrichtung ist der Dezellularisierungskammer 2 zur Dezellularisierung einer porcinen Aortenklappe 8 mit Hilfe der zusätzlichen Wirkung von Strömungskräften. Die Dezellularisierungskammer 2 besteht aus einem transparenten Hohlzylinder 9 aus Piacryl mit an den offenen Stirnseiten zentrisch und abdichtend fixierten Einsatzstücken 10 und 11 aus Teflon, die über Anschlussstutzen 12, 13 mit der Ringleitung 1 verbunden sind und jeweils einen in den Hohlzylinder 9 ragenden Befestigungsabschnitt 14, 15 mit radial am Umfang angeordneten Befestigungslöchern 16, 17 zum straffen Halten der Aortenklappe 8 unter Spannung an deren stirnseitigen Rändern aufweisen. Der Außendurchmesser der beiden Befestigungsabschnitte 14, 15 der Einsatzstücke 10, 11 entspricht etwa dem Durchmesser der Aortenklappe 8. Das in Strömungsrichtung hintere Einsatzstück 11 ist über einen Bundsteg 18 und eine erste Dichtung 27 an der Innenseite eines mit dem Hohlzylinder 9 verbundenen Ringsteges 20 verspannbar, und zwar mit Hilfe eines Schraubringes 21, dessen Innengewinde in ein Außengewinde am Einsatzstück 11 eingreift. Das Einsatzstück 10 weist einen Bund 22 auf, der an der Stirnfläche des Hohlzylinders 9 zur Anlage kommt und mit dieser unter Verwendung einer Schraubkappe 23 mit Innengwinde, das in ein Außengewinde am Hohlzylinder 9 eingreift, verspannbar ist. Zur flüssigkeitsdichten Befestigung ist eine zweite Dichtung 19 vorgesehen. Das der Dezellularisierungskammer nachgeschaltete Schlauchstück der Ringleitung 1 besteht aus flexiblem Material (Silikon), so dass die Durchströmung aufgrund der pulsierenden Pumpwiriung gewährleistet ist.

Aufgrund der zuvor beschriebenen Ausbildung und Anordnung der Einsatzstücke 10, 11 kann außerhalb des Hohlzylinders 9 eine entsprechend vorbereitere Aortenklappe 88 an den gegenüberliegenden Befestigungsabschnitten 14,15 der Einsatzstücke 10, 11 angenäht werden. In dieser Form wird die Aortenklappe 8 in den Hohlzylinder 9 eingebracht. Über ein Füll- und Ablassventil 24, 25 in der Ringleitung 1 oder einen der Anschlussstutzen 13, 14 wird zunächst Desoxycholsäure in die Dezellularisierungskammer 2 und die Ringleitung 1 eingebracht und danach die Membranpumpe 3 in Betrieb gesetzt, so dass ein pulsierender Desoxycholsäurestrom kontinuierlich über bzw. durch die Aortenklappe 8 fließt und aufgrund der mechanischen Einwirkung auf das Gewebe eine Vervollständigung der Ablösung und Entfernung der für den Empfänger der Herzklappe fremden Zellen bewirkt. Nach dem Ablassen der Desoxycholsäure wird physiologische Kochsalzlösung oder Phosphatpufferlösung eingebracht und bis zur restlosen Entfernung der Desoxycholsäure und toxischer Bestandteile gespült. An den Spülvorgang schließt sich eine Behandlung der Herzklappe 8 in Alkohol an, gefolgt von einem Spülschritt in Phosphatpufferlösung.

In der oben beschriebenen Vorrichtung erfolgen alle Behandlungsschritte des Dezellularisierungsverfahrens in einer pulsierenden Strömung des jeweiligen Mediums. Die Strömungsrichtung entspricht dabei etwa dem natürlichen Strömungsverlauf in implantiertem Zustand der Bioprothese. Zum Austausch der Medien werden die Füll- und Ablassventile 24, 25 benutzt, wobei für den Spülvorgang auch kontinuierlich frische Spüllösung zugeführt und verbrauchte Spüllösung abgeführt werden kann.

Wahlweise ist es auch möglich, einen oder mehrere Behandlungsschritte, gelöst von der Ringleitung und der Membranpumpe, ohne Durchströmung der Dezellularisierungskammer, die gegebenenfalls motorisch oder manuell gedreht wird, durchzuführen oder auch, wie eingangs erläutert, einzelne Behandlungsschritte außerhalb der Dezellularisierungskammer vorzunehmen.

### Bezugszeichenliste

- 1: Ringleitung
- 2: Dezellularisierungskammer
- 3: Blutpumpe / Membranpumpe
- 4: Ausgleichskammer
- 5: Schlauchleitung (für 3)
- 6: Auslaßventil
- 7: Einlaßventil
- 8: Aortenklappe (Bioprothese)
- 9: Hohlzylinder
- 10: erstes Einsatzstück (in Strömungsrichtung vorn)
- 11: zweites Einsatzstück (in Strömungsrichtung hinten)
- 12: Anschlußstutzen
- 13: Anschlußstutzen
- 14: Befestigungsabschnitt
- 15: Befestigungsabschnitt
- 16: Befestigungslöcher
- 17: Befestigungslöcher
- 18: Bundsteg von 11
- 19: erste Dichtung
- 20: Ringsteg von 9
- 21: Schraubring
- 22: Bund von 10
- 23: Überwurfschraubklappe
- 24: Füll- und Ablassventil
- 25: Füll- und Ablassventil
- 26: Membranventil

## Patentansprüche

1. Verfahren zur Dezellularisierung von allogenem und xenogenem Fremdmaterial mit Hilfe von biologischen Detergentien für die Herstellung von mit körpereigenen Zellen des Protheseempfängers beschichteten Bioprothesen, **dadurch gekennzeichnet, dass** das Fremdmaterial zur Zelläbtötung sowie zum Ummanteln der Zellen und zum Trennen der Verbindung mit der Gewebematrix zunächst mit Gallensäure und zum weiteren Abtöten der Zellen mit Alkohol behandelt wird und beiden Schritten ein Spülvorgang nachgeschaltet ist, wobei mindestens der letzte Spülvorgang in einem Medienstrom zur mechanischen Einwirkung auf die Gewebematrix und die Zellen aufgrund der Strömungskräfte durchgeführt wird.

2. Verfahren nach Anspruch 1, **dadurch gekennzeichnet, dass** der Medienstrom pulsierend ist.

3. Verfahren nach Anspruch 1, **dadurch gekennzeichnet, dass** zur Ablösung der Zellen mit Gallensäure eine die Zellen umhüllende Desoxycholsäure eingesetzt wird.

4. Verfahren nach Anspruch 3, **dadurch gekennzeichnet, dass** die Desoxycholsäure 1-2 %-ig ist.

5. Verfahren nach Anspruch 1, **dadurch gekennzeichnet, dass** der zum Abtöten der Zellen eingesetzte Alkohol 30 bis 60 %-ig ist.

6. Verfahren nach Anspruch 5, **dadurch gekennzeichnet, dass** der Alkohol 40 %-ig ist.

7. Verfahren nach Anspruch 1, **dadurch gekennzeichnet, dass** als Spülmedium Phosphatpufferlösung eingesetzt wird.

8. Verfahren nach Anspruch 7, **dadurch gekennzeichnet, dass** das in ruhendem Medium vorgenommene Spülen bei sich stufenweise verringernder Konzentration des Spülmediums durchgeführt wird.

9. Verfahren nach Anspruch 1 bis 7, **dadurch gekennzeichnet, dass** in ruhendem Medium der Zeitraum der Desoxycholsäurebehandlung bis zu 24 Stunden, der Behandlung in Alkohol etwa 20 Minuten und des Spülens etwa 60 Minuten umfasst.

10. Verfahren nach Anspruch 1 bis 7, **dadurch gekennzeichnet, dass** die Behandlung des Fremdmaterials in Desoxycholsäure und/oder in Alkohol und/oder der zwischengeschaltete Spülzyklus in einem pulsierenden Medienstrom etwa entsprechend den natürlichen Strömungsverhältnissen in dem betreffenden Organ vorgenommen wird.

11. Verfahren nach Anspruch 1 und 10, **dadurch gekennzeichnet, dass** die Geschwindigkeit der strömenden Medien variabel ist.

12. Verfahren nach einem der Ansprüche 1, 10 und 11, **dadurch gekennzeichnet, dass** der Spülmedienstrom kontinuierlich oder diskontinuierlich erneuert wird.

13. Verfahren nach Anspruch 11, **dadurch gekennzeichnet, dass** die Konzentration des Spülmittelstroms allmählich verringert wird.

14. Verfahren nach einem der Ansprüche 1 und 10 - 13, **dadurch gekennzeichnet, dass** das zu behandelnde Fremdmaterial in dem jeweiligen Medienstrom in Strömungsrichtung unter Vorspannung gehalten wird.

## Claims

1. A method for decellularizing allogenic and xenogenic foreign material using biodetergents to produce bioprostheses coated with endogenic cells from the recipient of the prosthesis, **characterized in that** the foreign material is initially treated with bile acid to kill the cells and to coat the cells as well as to separate the bond with the tissue matrix, then treated with alcohol to kill more cells, each of these steps being followed by a rinsing step and at least the last rinsing step being executed in a media flow so that the fluid forces act mechanically on the tissue matrix and the cells.

2. The method according to claim 1 wherein the media flow is pulsating.

3. The method according to claim 1, **characterized in that** a cell-encompassing deoxycholic acid is used to detach the cells with bile acid.

4. The method according to claim 3 wherein 1% to 2% deoxycholic acid is used.

5. The method according to claim 1 wherein 30% to 60% alcohol is used to kill the cells.

6. The method according to claim 5 wherein 40% alcohol is used.

7. The method according to claim 1 wherein a phosphate buffer solution is used as rinsing medium.

8. The method according to claim 7 wherein decreasing concentrations of the rinsing medium are used for rinsing with non-flowing medium.

9. The method according to claims 1 through 7 **characterized in that** the deoxycholic acid treatment in non-flowing medium takes up to 24 hours, alcohol treatment takes about 20 minutes, and rinsing takes about 60 minutes.

10. The method according to claims 1 through 7 **characterized in that** the treatment of the foreign material in deoxycholic acid and/or alcohol and/or the intermediate rinsing cycle are carried out in a pulsating medium flow that comes close to the natural flow conditions in the respective organ.

11. The method according to claims 1 and 10 wherein the speed of the flowing media can be varied.

12. The method according to claims 1, 10 and 11 **characterized in that** the flowing rinsing medium is replaced continuously or discontinuously.

13. The method according to claim 11, **characterized in that** the concentration of the detergent flow is gradually reduced.

14. The method according to any one of claims 1 and 10 through 13, **characterized in that** the foreign material to be treated is held in a preloaded state in the respective media flow.

## Revendications

1. Procédé de décellularisation de matière étrangère allogène ou xénogène à l'aide de détergents biologiques pour la production de bioprothèses recouvertes de cellules du propre corps du récepteur de prothèse, **caractérisé en ce que** la matière étrangère est tout d'abord traitée avec de l'acide biliaire pour dévitaliser les cellules ainsi que pour envelopper les cellules et pour séparer la liaison avec la matrice tissulaire et est traitée avec de l'alcool pour dévitaliser encore les cellules, et **en ce qu'**une opération de rinçage a lieu après les deux étapes, au moins la dernière opération de rinçage étant effectuée dans un courant de fluide pour agir mécaniquement sur la matrice tissulaire et sur les cellules en raison de la force de l'écoulement.

2. Procédé selon la revendication 1, **caractérisé en ce que** le courant de fluide est pulsatoire.

3. Procédé selon la revendication 1, **caractérisé en ce que** pour le détachement des cellules avec de l'acide biliaire, on utilise de l'acide désoxycholique entourant les cellules.

4. Procédé selon la revendication 3, **caractérisé en ce que** l'acide désoxycholique a une concentration entre 1 et 2 %.

5. Procédé selon la revendication 1, **caractérisé en ce que** l'alcool utilisé pour dévitaliser les cellules a une concentration de 30 à 60 %.

6. Procédé selon la revendication 5, **caractérisé en ce que** l'alcool a une concentration de 40 %.

7. Procédé selon la revendication 1, **caractérisé en ce qu'**on utilise une solution tampon au phosphate en tant que fluide de rinçage.

8. Procédé selon la revendication 7, **caractérisé en ce que** le rinçage entrepris dans le fluide au repos est effectué avec une concentration de fluide de rinçage diminuant graduellement.

9. Procédé selon la revendication 7, **caractérisé en ce que** dans le fluide au repos, le traitement à l'acide désoxycholique dure jusqu'à 24 heures, le traitement à l'alcool dure approximativement 20 minutes, et le rinçage dure approximativement 60 minutes.

10. Procédé selon les revendications 1 à 7, **caractérisé en ce que** le traitement de la matière étrangère dans l'acide désoxycholique et/ou dans l'alcool et/ou le cycle de rinçage intercalé est effectué dans un courant de fluide pulsatoire de manière qui correspond approximativement aux conditions d'écoulement naturelles dans l'organe correspondant.

11. Procédé selon les revendications 1 à 10, **caractérisé en ce que** la vitesse des fluides en écoulement est variable.

12. Procédé selon l'une des revendications 1, 10 et 11, **caractérisé en ce que** le courant de fluide de rinçage est renouvelé de manière continue ou discontinue.

13. Procédé selon la revendication 11, **caractérisé en ce que** la concentration du courant de produit de rinçage est réduite progressivement.

14. Procédé selon l'une des revendications 1 et 10 à 13, **caractérisé en ce que** la matière étrangère à traiter est maintenue sous précontrainte dans le courant de fluide respectif en direction d'écoulement.
